(19) Europäisches Patentamt
European Patent Office
Office européen des brevets

(11) Veröffentlichungsnummer: **0 561 252 A1**

(12) **EUROPÄISCHE PATENTANMELDUNG**

(21) Anmeldenummer: **93103710.5**

(51) Int. Cl.⁵: **C07D 215/227**, A61K 31/47

(22) Anmeldetag: **09.03.93**

(30) Priorität: **16.03.92 DE 4208304**

(43) Veröffentlichungstag der Anmeldung: **22.09.93 Patentblatt 93/38**

(84) Benannte Vertragsstaaten: **AT BE CH DE DK ES FR GB GR IE IT LI LU NL PT SE**

(71) Anmelder: **MERCK PATENT GmbH**
**Postfach, Frankfurter Strasse 250**
**D-64271 Darmstadt(DE)**

(72) Erfinder: **Mederski, Werner, Dr.**
**Am Ohlenberg 29**
**W-6106 Erzhausen(DE)**
Erfinder: **Dorsch, Dieter, Dr.**
**Königsberger Strasse 17A**
**W-6105 Ober-Ramstadt(DE)**
Erfinder: **Beier, Norbert, Dr.**
**Georgenhäuserstrasse 19**
**W-6107 Reinheim 5(DE)**
Erfinder: **Lues, Ingeborg, Dr.**
**Katharinenstrasse 2**
**W-6100 Darmstadt(DE)**
Erfinder: **Minck, Klaus-Otto, Dr.**
**Büchestrasse 8**
**W-6105 Ober-Ramstadt(DE)**
Erfinder: **Schelling, Pierre, Prof. Dr.**
**Bordenbergweg 17**
**W-6109 Mühltal(DE)**

(54) **2-Oxochinolinderivate als Angiotensin II-Antagonisten.**

(57) 2-Oxochinolinderivate der Formel I

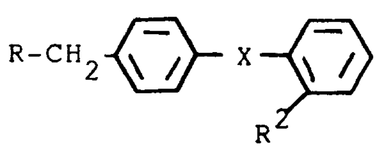

I

worin
R

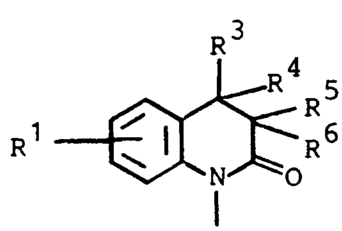

bedeutet und
$R^1$ bis $R^6$ und X die in Patentanspruch 1 angegebene Bedeutung haben,
sowie deren Salze zeigen angiotensin II-antagonistische Eigenschaften und wirken u.a. blutdrucksenkend.

EP 0 561 252 A1

Die Erfindung betrifft neue 2-Oxochinolinderivate der Formel I

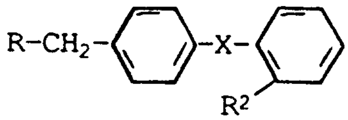

I

worin
R

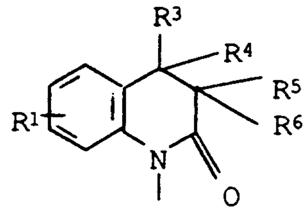

,

| | |
|---|---|
| $R^1$ | H, A, OA oder SA, |
| $R^2$ | H, COOH, COOA, CN, $NO_2$, $NH_2$, $NHCOR^7$, $NHSO_2R^7$ oder 1H-5-Tetrazolyl, |
| $R^3$ und $R^4$ | jeweils H oder A, |
| $R^5$ und $R^6$ | jeweils H, A, COOH, COOA, Cyan-alkyl, HOOC-alkyl, AOOC-alkyl, $H_2NCO$-alkyl, ANHCO-alkyl, $A_2NCO$-alkyl, Cyan-alkenyl, HOOC-alkenyl, AOOC-alkenyl, 1H-5-Tetrazolyl-alkyl, 2-Oxo-oxazolidinyl-alkyl, Ar-alkyl, AO-alkyl, ArO-alkyl, Ar-alkyl-O-alkyl, Formyl-alkyl, Oxo-alkyl, HOOC-oxo-alkyl, AOOC-oxo-alkyl, Oximino-alkyl, O-Alkyi-oximino-alkyl, HO-alkyl oder $R^8R^9N$-alkyl, |
| $R^3$ und $R^5$ | zusammen auch eine Bindung, |
| $R^7$ | Alkyl mit 1-6 C-Atomen, worin auch ein oder mehrere H-Atom(e) durch F ersetzt sein können, |
| $R^8$ | H oder A, |
| $R^9$ | H, A, A-CO, Ar-CO, COOA, $CONH_2$, CONHA, CONHAr, $CON(A)_2$ oder CONAAr, |
| X | fehlt, -NH-CO-, -CO-NH-, -O-CH (COOH) -, -NH-CH(COOH) -, -NA-CH(COOH)-, -CH=C-(COOH)-, -CH=C(CN)- oder -CH=C(1H-5-tetrazolyl)-, |
| A | Alkyl mit 1-6 C-Atomen, |
| Ar | eine unsubstituierte oder eine durch $R^7$, $OR^7$, Hal, COOH, COOA, CN, $NO_2$, $NH_2$, NHA, N-$(A)_2$, $NHCOR^7$, $NHSO_2R^7$ oder 1H-5-Tetrazolyl monosubstituierte Phenylgruppe und |
| Hal | F, Cl, Br oder I bedeuten und |

worin der "alkyl"-Teil oder "alkenyl"-Teil der genannten Gruppen jeweils bis zu 6 C-Atome enthält, sowie ihre Salze.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie angiotensin II-antagonistische Eigenschaften und können daher zur Behandlung der angiotensin II-abhängigen Hypertension, des Aldosteronismus und der Herzinsuffizienz sowie von Störungen des Zentralnervensystems eingesetzt werden. Diese Wirkungen können nach üblichen in vitro- oder in vivo-Methoden ermittelt werden, wie sie z.B. in der US-PS 4 880 804 und in der WO 91/14367 beschrieben sind, ferner von A.T. Chiu et al., J. Pharmacol. Exp. Therap. 250, 867-874 (1989), und von P.C. Wong et al., ibid. 252, 719-725 (1990; in vivo, an Ratten).

Die Verbindungen der Formel I können als Arzneimittelwirkstoffe in der Human- und Veterinärmedizin eingesetzt werden, insbesondere zur Prophylaxe und/oder Therapie von Herz-, Kreislauf- und Gefäßkrankheiten, vor allem von Hypertonie, Herzinsuffizienz und Hyperaldosteronismus.
ferner von Hypertrophie und Hyperplasie der Blutgefaße und des Herzens, Angina pectoris, Herzinfarkten, Schlaganfall, Restenosen nach Angioplastie oder By-pass-Operationen, Arteriosklerose, erhöhtem Augeninnendruck, Glaukomen, macularer Degeneration, Hyperurikämie, Nierenfunktionsstörungen, z.B. Nierenversagen, Nephropathia diabetica, Retinopathia diabetica, Psoriasis, angiotensin II-vermittelten Störungen in weiblichen Fortpflanzungsorganen, Wahrnehmungsstörungen, z.B. Demenz, Amnesie, Gedächtnisfunktionsstörungen, Angstzuständen, Depression und/oder Epilepsie.

Gegenstand der Erfindung sind die Verbindungen der Formel I und ihre Salze sowie ein Verfahren zur Herstellung dieser Verbindungen sowie ihrer Salze, dadurch gekennzeichnet, daß man

(a) eine Verbindung der Formel II

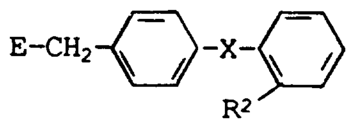

II

worin

E Cl, Br, I oder eine freie oder reaktionsfähig funktionell abgewandelte OH-Gruppe bedeutet und

$R^2$ und X die in Anspruch 1 angegebene Bedeutung haben,

mit einer Verbindung der Formel III

H-R III

worin

R die in Anspruch 1 angegebene Bedeutung hat,

umsetzt

oder

(b) zur Herstellung einer Verbindung der Formel I, worin

x NH-CO- oder -CO-NH- bedeutet,

eine Verbindung der Formel IV

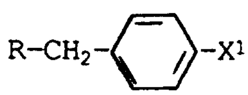

IV

worin

$X^1$ $NH_2$ oder COOH bedeutet und

R die in Anspruch 1 angegebene Bedeutung hat

oder ein reaktionsfähiges Derivat dieser Verbindung mit einer Verbindung der Formel V

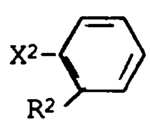

V

worin

$X^2$ COOH (falls $X^1$ $NH_2$ ist) oder $NH_2$ (falls $X^1$ COOH ist) bedeutet und

$R^2$ die in Anspruch 1 angegebene Bedeutung hat,

oder mit einem reaktionsfähigen Derivat dieser Verbindung umsetzt oder

(c) daß man eine Verbindung der Formel I aus einem ihrer funktionellen Derivate durch Behandeln mit einem solvolysierenden oder hydrogenolysierenden Mittel in Freiheit setzt,

und/oder daß man in einer Verbindung der Formel I einen oder mehrere Rest(e) R und/oder $R^2$ in einen oder mehrere andere Reste R und/oder $R^2$ umwandelt und/oder eine Base oder Säure der Formel I in eines ihrer Salze umwandelt.

Vor- und nachstehend haben die Reste bzw. Parameter R, $R^1$ bis $R^9$, X, A, Ar, Hal, E, $X^1$ und $X^2$ die bei den Formeln I, II, IV und V angegebenen Bedeutungen, falls nicht ausdrücklich etwas anderes angegeben ist.

In den vorstehenden Formeln hat A 1-6, vorzugsweise 1, 2, 3, oder 4 G-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3-oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl. Dementsprechend ist der Rest OA vorzugsweise Methoxy, weiterhin Ethoxy, Propoxy, Isopropoxy, Butoxy, Isobutoxy, sek.-Butoxy oder tert.-Butoxy, der Rest SA vorzugsweise Methylthio, weiterhin Ethylthio, Propylthio, Isopropylthio, Butylthio, Isobutylthio, sek.-Butylthio oder tert.-Butylthio.

Hal bedeutet vorzugsweise F, Cl oder Br, aber auch J.

Der Rest Ar ist vorzugsweise eine unsubstituierte Phenylgruppe, weiterhin bevorzugt eine in o-Stellung substituierte, aber auch in m- oder p-Stellung substituierte Phenylgruppe. Bevorzugte Substituenten sind COOH, COOA, $NO_2$, $NH_2$ und $N(A)_2$. Dementsprechend ist Ar bevorzugt Phenyl, (insbesondere) o-, m- oder p-Carboxyphenyl, (insbesondere) o-, m- oder p-Methoxycarbonylphenyl, (insbesondere) o-, m- oder p-Ethoxycarbonylphenyl, (insbesondere) o-, m- oder p-Nitrophenyl, (insbesondere) o-, m- oder p-Aminophenyl, (insbesondere) o-, m- oder p-Dimethylaminophenyl, (insbesondere) o-, m- oder p-Diethylaminophenyl, weiterhin bevorzugt o-, m- oder p-Tolyl, o-, m- oder p-Trifluormethylphenyl, o-, m- oder p-Methoxyphenyl, o-, m- oder p-Fluorphenyl, o-, m- oder p-Chlorphenyl, o-, m- oder p-Bromphenyl, o-, m- oder p-Iodphenyl, o-, m- oder p-Cyanphenyl, o-, m- oder p-Methylaminophenyl, o-, m- oder p-Acetamidophenyl, o-, m- oder p-Trifluoracetamidophenyl, o-, m- oder p-Methylsulfonamidophenyl, o-, m- oder p-Trifluormethylsulfonamidophenyl, o-, m- oder p-(1H-5-Tetrazolyl)-phenyl.

R ist ein von 1,2-Dihydrochinolin oder ein von 1,2,3,4-Tetrahydrochinolin abgeleiteter Rest, genauer:

(a) 1,2-Dihydro-2-oxo-3-$R^6$-4-$R^4$-5-(oder -6- oder -7- oder -8-)-$R^1$-chinolyl (falls $R^3$ und $R^5$ zusammen eine Bindung bedeuten),

(b) 1,2,3,4-Tetrahydro-2-oxo-3-$R^5$-3-$R^6$-4-$R^3$-4-$R^4$-5-(oder -6-oder -7- oder -8-)-$R^1$-chinolyl (falls $R^3$ und $R^5$ getrennt voneinander jeweils einen der angegebenen Reste bedeuten).

Dementsprechend umschließen die Verbindungen der Formel I solche der Formeln Ia und Ib, worin R die jeweils unter (a) oder (b) angegebene Bedeutung hat. Die Verbindungen der Formel Ia sind bevorzugt.

Der Rest $R^1$ ist vorzugsweise H oder A, insbesondere Methyl oder Ethyl.

Ein von H verschiedener Rest $R^1$ steht vorzugsweise in der 7- oder 8-Stellung des Chinolinrings.

Der Rest $R^2$ ist vorzugsweise CN, ferner bevorzugt 1H-5-Tetrazolyl, COOH, $COOCH_3$, $COOC_2H_5$ oder $NHSO_2CF_3$.

Die Reste $R^3$ und $R^4$ bedeuten jeweils vorzugsweise H oder Methyl. Weiterhin bedeuten $R^3$ und $R^5$ bevorzugt zusammen eine Bindung.

Von den Resten $R^5$ und $R^6$ ist der eine vorzugsweise H; der andere bedeutet bevorzugt ebenfalls H oder A (insbesondere $CH_3$), COOH, COOA (insbesondere Methoxycarbonyl, Ethoxycarbonyl), Cyanalkyl (insbesondere Cyanmethyl, 2-Cyanethyl, 3-Cyanpropyl), Carboxyalkyl (insbesondere Carboxymethyl, 2-Carboxyethyl, 3-Carboxypropyl), AOOC-alkyl (insbesondere Methoxycarbonylmethyl, Ethoxycarbonylmethyl, 2-Methoxycarbonyl-ethyl, 2-Ethoxycarbonyl-ethyl), Carbamoylalkyl (insbesondere Carbamoylmethyl, 2-Carbamoyl-ethyl), N-Alkylcarbamoylalkyl [insbesondere N-Methyl-carbamoyl-methyl, N-Ethyl-carbamoyl-methyl, 2-(N-Methyl-carbamoyl)-ethyl, 2-(N-Ethyl-carbamoyl-ethyl], N,N-Dialkyl-carbamoylalkyl [insbesondere N,N-Dimethyl-carbamoyl-methyl, N,N-Diethylcarbamoyl-methyl, 2-(N,N-Dimethyl-carbamoyl)-ethyl, 2-(N,N-Diethyl-carbamoyl)-ethyl], Cyanalkenyl (insbesondere 3-Cyan-2-propen-1-yl), Carboxyalkenyl (insbesondere 3-Carboxy-2-propen-1-yl), Alkoxycarbonyl-alkenyl (insbesondere 3-Methoxycarbonyl-2-propen-1-yl, 3-Ethoxycarbonyl-2-propen-1-yl),1H-5-Tetrazolyl-alkyl [insbesondere 1H-5-Tetrazolyl-methyl, 2-(1H-5-Tetrazolyl)-ethyl, 3-(1H-5-Tetrazolyl)-propyl], 2-Oxo-oxazolidinyl-alkyl [insbesondere 2-Oxo-oxazolidinyl-methyl, 2-(2-Oxo-oxazolidinyl)-ethyl], Ar-alkyl [insbesondere Benzyl, 1- oder 2-Phenylethyl, 1-, 2- oder 3-Phenylpropyl, 1-, 2-, 3- oder 4-Phenylbutyl, o-, m- oder p-Fluorbenzyl, (bevorzugt) o-, m- oder p-Chlorbenzyl, o-, m- oder p-Brombenzyl, o-, m- oder p-Methylbenzyl, o-, m- oder p-Trifluormethylbenzyl, o-, m- oder p-Methoxycarbonylbenzyl, o-, m- oder p-Ethoxycarbonylbenzyl, (bevorzugt) o-, m- oder p-Cyanbenzyl, o-, m- oder p-Carboxybenzyl, o-, m- oder p-Nitrobenzyl, (bevorzugt) o-, m- oder p-Aminobenzyl, o-, m- oder p-Dimethylaminobenzyl, (bevorzugt) o-, m- oder p-(1H-5-Tetrazoly)-benzyl], Alkoxy-alkyl (insbesondere Methoxymethyl, Ethoxymethyl, 2-Methyloxy-ethyl, 2-Ethoxyethyl), Ar-oxy-alkyl (insbesondere Phenoxy-methyl, 2-Phenoxy-ethyl), Ar-alkyl-oxy-alkyl (insbesondere Benzyloxy-methyl, 2-Benzyloxy-ethyl), Formyl-alkyl (insbesondere Formyl-methyl, 2-Formyl-ethyl), Oxo-alkyl(insbesondere 2-Oxo-propyl, 2- oder 3-Oxobutyl, 3,3-Dimethyl-2-oxo-butyl), Carboxy-oxo-alkyl (insbesondere 2-Carboxy-2-oxo-ethyl), Alkoxycarbonyl-oxo-alkyl (insbesondere 2-Methoxycarbonyl-2-oxo-ethyl, 2-Ethoxycarbonyl-2-oxo-ethyl), Oximino-alkyl (insbesondere 2-Oximino-propyl, 2- oder 3-Oximino-butyl, 3,3-Dimethyl-2-oximino-butyl), O-Alkyl-oximino-alkyl [insbesondere 2-(O-Methyl-oximino)-propyl, 2-(O-Ethyl-oximino)-propyl, 2- oder 3-(O-Methyl-oximino)-butyl, 3,3-Dimethyl-2-(O-methyl-oximino)-butyl], Hydroxy-alkyl (insbesondere Hydroxy-methyl, 2-Hydroxy-ethyl), Amino-alkyl (insbesondere Aminomethyl, 2-Amino-ethyl), Alkylamino-alkyl (insbesondere Methylamino-methyl, Ethylamino-methyl, 2-Methylamino-ethyl, 2-Ethylamino-ethyl), Dialkylamino-alkyl (insbesondere

Dimethylamino-methyl, Diethylamino-methyl, 2-Dimethylamino-ethyl, 2-Diethylamino-ethyl), Alkanoylamino-alkyl (insbesondere Acetamido-methyl, Propionamido-methyl, 2-Acetamido-ethyl), Aroylamino-alkyl (insbesondere Benzamido-methyl, 2-Benzamido-ethyl), Alkoxycarbonylamino-alkyl (insbesondere Methoxycarbonylamino-methyl, Ethoxycarbonylamino-methyl, 2-Methoxycarbonylamino-ethyl, 2-Ethoxycarbonylamino-ethyl), Ureido-alkyl (insbesondere Ureido-methyl, 2-Ureido-ethyl), N-Alkylureido-alkyl (insbesondere N-Methylureido-methyl, N-Ethylureido-methyl, 2-N-Methylureido-ethyl, 2-N-Ethylureido-ethyl), N,N-Dialkylureido-alkyl (insbesondere N,N-Dimethylureido-methyl, N,N-Diethylureido-ethyl, 2-N,N-Dimethylureido-ethyl, 2-N,N-Diethylureido-ethyl), N-Ar-ureido-alkyl (insbesondere N-Phenylureido-methyl, 2-N-Phenylureido-ethyl), N-Alkyl-N-Ar-ureido-alkyl (insbesondere N-Methyl-N-phenylureido-methyl, N-Ethyl-H-phenylureido-methyl).

Der Rest $R^7$ ist vorzugsweise Trifluormethyl, ferner bevorzugt A wie Methyl oder Ethyl sowie Fluormethyl, Difluormethyl, Pentafluorethyl oder Heptafluorpropyl.

Der Rest $R^8$ ist vorzugsweise H, ferner bevorzugt Methyl oder Ethyl.

Der Rest $R^9$ ist Vorzugsweise H, A (insbesondere Methyl oder Ethyl) oder A-CO (insbesondere Acetamido).

Der Rest X fehlt vorzugsweise.

Der "alkyl"-Teil der genannten Gruppen ist vorzugsweise $-CH_2$-oder $-CH_2CH_2-$, ferner bevorzugt $-CH(CH_3)-$, $-(CH_2)_3-$, $-(CH_2)_4-$, $-(CH_2)_5-$ oder $-(CH_2)_6-$, der "alkenyl"-Teil vorzugsweise $-CH_2-CH=CH$.

Die Verbindungen der Formel I können ein oder mehrere chirale Zentren besitzen und daher in Verschiedenen - optisch-aktiven oder optisch-inaktiven - Formen Vorkommen. Die Formel I umschließt alle diese Formen.

Dementsprechend sind Gegenstand der Erfindung insbesondere diejenigen Verbindungen der Formel I, in denen mindestens einer der genannten Reste eine der Vorstehend angegebenen bevorzugten Bedeutungen hat. Einige bevorzugte Gruppen von Verbindungen können durch die folgenden Teilformeln Ic, Id, Ie, Iac, Iad, Iae, Ibc, Ibd, Ibe, Icd, Ice, Ide, Iacd, Ibcd, Iace, Ibce, Iade, Ibde, Icde, Iacde und Ibcde ausgedrückt werden, die den Formeln I sowie Ia und Ib entsprechen und worin die nicht näher bezeichneten Reste die bei den Formeln I sowie Ia und Ib angegebenen Bedeutungen haben:

Verbindungen der Formel Ic sowie Iac und Ibc: diese entsprechen den Formeln I sowie Ia und Ib, zusätzlich bedeutet in ihnen jedoch $R^1$ H, Methyl oder Ethyl.

Verbindungen der Formeln Id sowie Iad, Ibd, Icd, Iacd und Ibcd: diese entsprechen den Formeln I sowie Ia, Ib, Ic, Iac und Ibc, zusätzlich bedeutet in ihnen jedoch $R^2$ CN oder (bevorzugt) 1H-5-Tetrazolyl.

Verbindungen der Formeln Ie sowie Iae, Ibe, Ice, Ide, Iace, Ibce, Iade, Ibde, Icde, Iacde, Ibcde: diese entsprechen den Formeln I, Ia, Ib, Ic, Id, Iac, Ibc, Iad, Ibd, Icd, Iacd und Ibcd, zusätzlich bedeutet jedoch in ihnen $R^3$ H oder $CH_3$ und $R^4$ H.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; insbesondere aber in der EP-A2-0 430 709 und in der US-PS 4 880 804) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können vorzugsweise erhalten werden, indem man Verbindungen der Formel II mit Verbindungen der Formel III umsetzt.

In den Verbindungen der Formel II bedeutet E Vorzugsweise Cl, Br, I oder eine reaktionsfähig funktionell abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy).

Die Umsetzung von II mit III erfolgt zweckmäßig, indem man zunächst III durch Behandeln mit einer Base in ein Salz umwandelt, z.B. mit einem Alkalimetallalkoholat wie $CH_3ONa$ oder K-tert.-Butylat in einem Alkohol wie $CH_3OH$ oder in einem Amid wie Dimethylformamid (DMF) oder mit einem Alkalimetallhydrid wie NaH oder einem Alkalimetallalkoholat in DMF, und dieses dann in einem inerten Lösungsmittel, z.B. einem Amid wie DMF oder Dimethylacetamid oder einem Sulfoxid wie Dimethylsulfoxid (DMSO), mit II umsetzt, zweckmäßig bei Temperaturen zwischen -20 und 100°, vorzugsweise zwischen 10 und 30°. Als Basen eignen sich auch Alkalimetallcarbonate wie $Na_2CO_3$ oder $K_2CO_3$ oder Alkalimetall-hydrogencarbonate wie $NaHCO_3$ oder $KHCO_3$.

Die Ausgangsstoffe, insbesondere diejenigen der Formel III, sind teilweise bekannt. Falls sie nicht bekannt sind, können sie nach bekannten Methoden in Analogie zu bekannten Stoffen hergestellt werden.

Säureamide der Formel I (X = -NH-CO- oder -CO-NH-) sind ferner erhältlich durch Umsetzung Von Verbindungen der Formel IV (oder ihrer reaktionsfähigen Derivate) mit Verbindungen der Formel V (oder ihrer reaktionsfähigen Derivate).

Als reaktionsfähige Derivate der Carbonsäuren der Formeln IV und V ($X^1$ bzw. $X^2$ = COOH) eignen sich vorteilhaft die entsprechenden Chloride, Bromide oder Anhydride. Die Umsetzung erfolgt zweckmäßig in Gegenwart eines inerten Lösungsmittels, z.B. eines halogenierten Kohlenwasserstoffs wie Dichlormethan, Chloroform, Trichlorethan oder 1,2-Dichlorethan oder eines Ethers wie Tetrahydrofuran (THF) oder Dioxan, bei Temperaturen zwischen 0 und 150°, vorzugsweise zwischen 20 und 80°. Setzt man Säurehalogenide um, so empfiehlt sich der Zusatz einer Base, z.B. eines tertiären Amins wie Triethylamin, Pyridin oder 4-Dimethylaminopyridin.

Man kann ferner eine Verbindung der Formel I aus einem ihrer funktionellen Derivate durch Behandeln mit einem solvolysierenden (z.B. hydrolysierenden) oder hydrogenolysierenden Mittel in Freiheit setzen.

So ist es möglich, nach einer der angegebenen Methoden eine Verbindung herzustellen, die der Formel I entspricht, aber an Stelle einer 5-Tetrazolylgruppe eine in 1-Stellung funktionell abgewandelte (durch eine Schutzgruppe geschützte) 5-Tetrazolylgruppe enthält. Als Schutzgruppen eignen sich beispielsweise: Triphenylmethyl, abspaltbar mit HCl oder Ameisensäure in einem inerten Lösungsmittel oder Lösungsmittelgemisch, z.B. Methanol oder Ether/Dichlormethan/Methanol; 2-Cyanethyl, abspaltbar mit NaOH in Wasser/THF; p-Nitrobenzyl, äbspaltbar mit $H_2$/Raney-Nickel in Ethanol (vgl. EP-A2-0 291 969).

Es ist ferner möglich, eine Verbindung der Formel I in eine andere Verbindung der Formel I umzuwandeln, indem man einen oder mehrere der Reste R und/oder $R^2$ in andere Reste R und/oder $R^2$ umwandelt, z.B. indem man Nitrogruppen (beispielsweise durch Hydrierung an Raney-Nickel oder Pd-Kohle in einem inerten Lösungsmittel wie Methanol oder Ethanol) zu Aminogruppen reduziert und/oder freie Amino- und/oder Hydroxygruppen funktionell abwandelt und/oder funktionell abgewandelte Amino- und/oder Hydroxygruppen durch Solvolyse oder Hydrogenolyse freisetzt und/oder Halogenatome (z.B. durch Reaktion mit Kupfer(I)cyanid) durch CN-Gruppen ersetzt und/oder Nitrilgruppen zu COOH-Gruppen hydrolysiert oder mit Derivaten der Stickstoffwasserstoffsäure, z.B. Natriumazid in N-Methylpyrrolidon oder Trimethylzinnazid in Toluol, zu Tetrazolylgruppen umsetzt.

So kann man beispielsweise freie Aminogruppen in üblicher Weise mit einem Säurechlorid oder -anhydrid acylieren oder freie Hydroxy- und/oder NH-Gruppen mit einem unsubstituierten oder substituierten Alkyl- oder Ar-alkylhalogenid oder mit Aldehyden wie Formaldehyd in Gegenwart eines Reduktionsmittels wie $NaBH_4$ oder Ameisensäure alkylieren, zweckmäßig in einem inerten Lösungsmittel wie Dichlormethan oder THF und/oder in Gegenwart einer Base wie Triethylamin oder Pyridin bei Temperaturen zwischen -60 und +30°.

Gewünschtenfalls kann in einer Verbindung der Formel I eine funktionell abgewandelte Amino- und/oder Hydroxygruppe durch Solvolyse oder Hydrogenolyse nach üblichen Methoden in Freiheit gesetzt werden. So kann z. B. eine Verbindung der Formel I, die eine $NHCOR^7$- oder eine COOA-Gruppe enthält, in die entsprechende Verbindung der Formel I umgewandelt werden, die stattdessen eine $NH_2$- oder eine COOH-Gruppe enthält. Estergruppen können z.B. mit NaOH oder KOH in Wasser, Wasser-THF oder Wasser-Dioxan bei Temperaturen zwischen 0 und 100° verseift werden.

Umsetzung von Nitrilen der Formel 1 ($R^2$ = CN oder $R^5$ oder R6 = Cyanalkyl) mit Derivaten der Stickstoffwasserstoffsäure führt zu Tetrazolen der Formel I ($R^2$ = 1H-5-Tetrazolyl und/oder $R^5$ oder $R^6$ = 1H-5-Tetrazolyl-alkyl). Bevorzugt verwendet man Trialkylzinnazide wie Trimethylzinnazid in einem inerten Lösungsmittel, z.B. einem aromatischen Kohlenwasserstoff wie Toluol bei Temperaturen zwischen 20 und 150°, vorzugsweise zwischen 80 und 140°, oder Natriumazid in N-Methylpyrrolidon bei Temperaturen zwischen etwa 100 und 200°.

Weiterhin kann man ein Keton der Formel I ($R^5$ und/oder $R^6$ = z.B. Oxoalkyl) durch Reaktion mit Hydroxylamin oder O-Alkylhydroxylamin in das entsprechende Oxim oder O-Alkyl-oxim umwandeln, zweckmäßig in einem inerten Lösungsmitteln bei 10-30°.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern, So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Andererseits können Verbindungen der Formel I, die COOH- oder Tetrazolylgruppen enthalten, mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden. Die Kaliumsalze der Tetrazolylderivate sind besonders bevorzugt.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff-(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vor zugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgas oder Treibgasgemisch (z.B. Kohlenwasserstoffen wie Propan oder Butan oder Fluorkohlenwasserstoffen wie Heptafluorpropan) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine, Diuretika, Antiphlogistika.

Die erfindungsgemäßen Substanzen werden in der Regel in Analogie zu anderen bekannten, im Handel befindlichen Präparaten, insbesondere aber in Analogie zu den in der US-PS 4 880 804 beschriebenen Verbindungen verabreicht, vorzugsweise in Dosierungen zwischen etwa 1 mg und 1 g, insbesondere zwischen 50 und 500 mg pro Dosierungseinheit. Die tägliche Dosierung liegt Vorzugsweise zwischen etwa 0,1 und 100 mg/kg, insbesondere 1 und 50 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und/oder durch Kristallisation. Rf = Rf an Kieselgel (dünnschichtchromatographisch; Laufmittel: Ethylacetat/Hexan 9:1)

## Beispiel 1

a) Eine Lösung von 20,3 g 7-Ethyl-1,2,3,4-tetrahydro-4,4-dimethyl-2-oxo-chinolin [F. 120°; erhältlich durch Reaktion von m-Ethylanilin mit 3,3-Dimethylacryloylchlorid in Toluol/Dichlormethan zu N-(3,3-Dimethylacryloyl)-m-ethylanilin (F. 90°) und Cyclisierung mit $AlCl_3$ bei 60-100°] in 700 ml DMF wird bei 20° mit 12,5 g K-tert.-Butoxid versetzt. Man rührt 45 Minuten, tropft dann eine Lösung von 30,5 g 4-Brommethyl-2'-cyan-biphenyl in 300 ml DMF hinzu, rührt über Nacht, dampft ein, arbeitet wie üblich auf und erhält 1-(2'-Cyan-biphenylyl-4-methyl)-7-ethyl-1,2,3,4-tetrahydro-4,4-dimethyl-2-oxo-chinolin, F. 171-172°.

Analog erhält man die nachstehenden 1-(2'-Cyan-biphenyl-yl-4-methyl)-2-oxo-chinoline:
aus 1,2-Dihydro-2-oxo-chinolin (F. 198°): 1,2-Dihydro-, F. 175°;

aus 1,2,3,4-Tetrahydro-2-oxo-chinolin (F. 168°):
1,2,3,4-Tetrahydro-, F. 97°;
aus 1,2,3,4-Tetrahydro-4,4-dimethyl-2-oxo-chinolin (F. 114-115°):
1,2,3,4-Tetrahydro-4,4-dimethyl-, F. 139°;
aus 7-Ethyl-1,2-dihydro-4-methyl-2-oxo-chinolin [F. 180°; erhältlich durch Reaktion von 3-Ethylanilin mit 2,2,6-Trimethyl-1,3-dioxin-4-on bei 120° zu 3-Ethyl-N-(3-oxobutyryl)-anilin (Rf 0,52) und Cyclisierung mit $H_2SO_4$ bei 20°]:
7-Ethyl-1,2-dihydro-4-methyl-;
aus 7-Ethyl-1,2-dihydro-4-methyl-3-p-nitrobenzyl-2-oxo-chinolin [F. 284°; erhältlich durch Reaktion von 3-Ethyl-N-(3-oxobutyryl)-anilin mit p-Nitrobenzylchlorid zu 3-Ethyl-N-(2-p-nitrobenzyl-3-oxo-butyryl)-anilin und Cyclisierung]:
7-Ethyl-1,2-dihydro-4-methyl-3-p-nitrobenzyl-, F. 171°;
aus 3,7-Diethyl-1,2-dihydro-4-methyl-2-oxo-chinolin:
3,7-Diethyl-1,2-dihydro-4-methyl-;
aus 3-Carboxy-7-ethyl-1,2-dihydro-4-methyl-2-oxo-chinolin:
3-Carboxy-7-ethyl-1,2-dihydro-4-methyl-;
aus 7-Ethyl-1,2-dihydro-3-methoxycarbonyl-4-methyl-2-oxo-chinolin:
7-Ethyl-1,2-dihydro-3-methoxycarbonyl-4-methyl-;
aus 3-Cyanmethyl-7-ethyl-1,2-dihydro-4-methyl-2-oxo-chinolin:
3-Cyanmethyl-7-ethyl-1,2-dihydro-4-methyl-;
aus 3-Carboxymethyl-7-ethyl-1,2-dihydro-4-methyl-2-oxo-chinolin:
3-Carboxymethyl-7-ethyl-1,2-dihydro-4-methyl-;
aus 7-Ethyl-1,2-dihyöro-3-methoxycarbonylmethyl-4-methyl-2-oxo-chinolin (F. 209°):
7-Ethyl-1,2-dihydro-3-methoxycarbonylmethyl-4-methyl-, F. 115°;
aus 7-Ethyl-1,2-dihydro-3-carbamoylmethyl-4-methyl-2-oxo-chinolin (F. 289°):
7-Ethyl-1,2-dihydro-3-carbamoylmethyl-4-methyl-, Dihydrat, F. 253°;
aus 7-Ethyl-1,2-dihydro-4-methyl-3-N,N-dimethylcarbamoylmethyl-2-oxo-chinolin (F. 232°):
7-Ethyl-1,2-dihydro-4-methyl-3-N,N-dimethylcarbamoylmethyl-, Sesquihydrat, F. 193°;
aus 7-Ethyl-1,2-dihydro-4-methyl-3-N,N-diethylcarbamoylmethyl-2-oxo-chinolin (F. 228°):
7-Ethyl-1,2-dihydro-4-methyl-3-N,N-diethylcarbamoylmethyl-, F. 188°;
aus 3-(3-Carboxy-2-propen-1-yl)-7-ethyl-1,2-dihydro-4-methyl-2-oxo-chinolin:
3-(3-Carboxy-2-propen-1-yl)-7-ethyl-1,2-dihydro-4-methyl-;
aus 7-Ethyl-1,2-dihydro-3-(3-methoxycarbonyl-2-propen-1-yl)-4-methyl-2-oxo-chinolin:
7-Ethyl-1,2-dihydro-3-(3-methoxycarbonyl-2-propen-1-yl)-4-methyl-;
aus 7-Ethyl-1,2-dihydro-4-methyl-2-oxo-3-(1H-5-tetrazolylmethyl)-chinolin:
7-Ethyl-1,2-dihydro-4-methyl-3-(1H-5-tetrazolylmethyl)-;
aus 7-Ethyl-1,2-dihydro-4-methyl-2-oxo-3-(2-oxo-5-oxazolidinylmethyl)-chinolin:
7-Ethyl-1,2-dihydro-4-methyl-3-(2-oxo-5-oxazolidinylmethyl)-;
aus 7-Ethyl-1,2-dihydro-4-methyl-3-o-nitrobenzyl-2-oxo-chinolin:
7-Ethyl-1,2-dihydro-4-methyl-3-o-nitrobenzyl-;
aus 7-Ethyl-1,2-dihydro-4-methyl-3-m-nitrobenzyl-2-oxo-chinolin:
7-Ethyl-1,2-dihydro-4-methyl-3-m-nitrobenzyl-;
aus 3-o-Carboxybenzyl-7-ethyl-1,2-dihydro-4-methyl-2-oxo-chinolin:
3-o-Carboxybenzyl-7-ethyl-1,2-dihydro-4-methyl-;
aus 7-Ethyl-1,2-dihydro-3-o-methoxycarbonylbenzyl-4-methyl-2-oxo-chinolin:
7-Ethyl-1,2-dihydro-3-o-methoxycarbonylbenzyl-4-methyl-;
aus 7-Ethyl-1,2-dihydro-3-p-methoxycarbonylbenzyl-4-methyl-2-oxo-chinolin (F. 236°):
7-Ethyl-1,2-dihydro-3-p-methoxycarbonylbenzyl-4-methyl-, Trihydrat, F. 98°;
aus 7-Ethyl-1,2-dihydro-4-methyl-3-o-dimethylaminobenzyl-2-oxo-chinolin:
7-Ethyl-1,2-dihydro-4-methyl-3-o-dimethylaminobenzyl-;
aus 7-Ethyl-1,2-dihydro-3-methoxymethyl-4-methyl-2-oxo-chinolin:
7-Ethyl-1,2-dihydro-3-methoxymethyl-4-methyl-;
aus 3-Benzyloxymethyl-7-ethyl-1,2-dihydro-4-methyl-2-oxo-chinolin:
3-Benzyloxymethyl-7-ethyl-1,2-dihydro-4-methyl-;
aus 7-Ethyl-3-formylmethyl-1,2-dihydro-4-methyl-2-oxo-chinolin:
7-Ethyl-3-formylmethyl-1,2-dihydro-4-methyl-;
aus 7-Ethyl-1,2-dihydro-4-methyl-2-oxo-3-(2-oxo-propyl)-chinolin:
7-Ethyl-1,2-dihydro-4-methyl- -3-(2-oxo-propyl)-;

aus 7-Ethyl-1,2-dihydro-4-methyl-3-(3,3-dimethyl-2-oxo-butyl)-2-oxo-chinolin:
7-Ethyl-1,2-dihydro-4-methyl-3-(3,3-dimethyl-2-oxo-butyl)-;
aus 3-(2-Carboxy-2-oxo-ethyl)-7-ethyl-1,2-dihydro-4-methyl-2-oxo-chinolin:
3-(2-Carboxy-2-oxo-ethyl)-7-ethyl-1,2-dihydro-4-methyl-;
aus 3-(2-Ethoxycarbonyl-2-oxo-ethyl)-7-ethyl-1,2-dihydro-4-methyl-2-oxo-chinolin:
3-(2-Ethoxycarbonyl-2-oxo-ethyl)-7-ethyl-1,2-dihydro-4-methyl-;
aus 7-Ethyl-1,2-dihydro-4-methyl-3-(2-oximino-propyl)-2-oxo-chinolin:
7-Ethyl-1,2-dihydro-4-methyl-3-(2-oximino-propyl)-;
aus 7-Ethyl-1,2-dihydro-4-methyl-3-(3,-dimethyl-2-oximino-butyl)-2-oxo-chinolin:
7-Ethyl-1,2-dihydro-4-methyl-3-(3,3-dimethyl-2-oximino-butyl)-;
aus 7-Ethyl-1,2-dihydro-4-methy-3-(2-O-methyl-oximinopropyl)-2-oxo-chinolin:
7-Ethyl-1,2-dihydro-4-methyl-3-(2-O-methyl-oximinopropyl)-;
aus 7-Ethyl-1,2-dihydro-4-methyl-3-(3,3-dimethyl-2-O-methyl-oximino-butyl)-2-oxo-chinolin:
7-Ethyl-1,2-dihydro-4-methyl-3-(3,3-dimethyl-2-O-methyl-oximino-butyl)-;
aus 7-Ethyl-1,2-dihydro-3-hydroxymethyl-4-methyl-2-oxo-chinolin:
7-Ethyl-1,2-dihydro-3-hydroxymethyl-4-methyl-;

b) Ein Gemisch von 3,94 g der nach (a) erhaltenen Verbindung, 20,6 g Trimethylzinnazid und 200 ml Toluol wird 24 Std. gekocht und dann eingedampft. Man nimmt den Rückstand in 100 ml methanolischer HCl auf, rührt 2 Stunden bei 20°, dampft ein, arbeitet wie üblich auf und erhält 7-Ethyl-1,2,3,4-tetrahydro-4,4-dimethyl-2-oxo-1-[2'-(1H-5-tetrazolyl)-biphenylyl-4-methyl]-chinolin, Trihydrat, F. 200°.

Analog erhält man die nachstehenden 2-Oxo-1-[2'-(1H-5-tetrazolyl)-biphenylyl-4-methyl]-chinoline:
1,2-Dihydro-, F. 265°
1,2,3,4-Tetrahydro-, F. 236°
1,2,3,4-Tetrahydro-4,4-dimethyl-, F. 176°
7-Ethyl-1,2-dihydro-4-methyl-, F. 260°
7-Ethyl-1,2-dihydro-4-methyl-3-p-nitrobenzyl-,
Tetrahydrat, F. 229°
3,7-Diethyl-1,2-dihydro-4-methyl-
3-Carboxy-7-ethyl-1,2-dihydro-4-methyl-
7-Ethyl-1,2-dihydro-3-methoxycarbonyl-4-methyl-
3-Cyanmethyl-7-ethyl-1,2-dihydro-4-methyl- [daneben die entsprechende 3-(1H-5-Tetrazolyl-methyl)-verbindung]
3-Carboxymethyl-7-ethyl-1,2-dihydro-4-methyl-, Trihydrat, F. 262°
7-Ethyl-1,2-dihydro-3-methoxycarbonylmethyl-4-methyl-, F. 215°
7-Ethyl-1,2-dihydro-3-carbamoylmethyl-4-methyl-, F. 262°
7-Ethyl-1,2-dihydro-4-methyl-3-N,N-dimethylcarbamoylmethyl-, Tetrahydrat, F. 225°
7-Ethyl-1,2-dihydro-4-methyl-3-N,N-diethylcarbamoylmethyl, F. 165°
3-(3-Carboxy-2-propen-1-yl)-7-ethyl-1,2-dihydro-4-methyl-7-Ethyl-1,2-dihydro-3-(3-methoxycarbonyl-2-propen-yl)-4-methyl-
7-Ethyl-1,2-dihydro-4-methyl-3-(1H-5-tetrazolyl-methyl)-
7-Ethyl-1,2-dihydro-4-methyl-3-(2-oxo-5-oxazolidinylmethyl)-
7-Ethyl-1,2-dihydro-4-methyl-3-onitrobenzyl-
7-Ethyl-1,2-dihydro-4-methyl-3-m-nitrobenzyl-
3-o-Carboxybenzyl-7-ethyl-1,2-dihydro-4-methyl-
7-Ethyl-1,2-dihydro-3-o-methoxycarbonylbenzyl-4-methyl-
7-Ethyl-1,2-dihydro-4-methyl-3-o-dimethylaminobenzyl-
7-Ethyl-1,2-dihydro-3-methoxymethyl-4-methyl-
3-Benzyloxymethyl-7-ethyl-1,2-dihydro-4-methyl-
7-Ethyl-3-formylmethyl-1-2-dihydro-4-methyl-
7-Ethyl-1,2-dihydro-4-methyl-3-(2-oxo-propyl)-
7-Ethyl-1,2-dihydro-4-methyl-3-(3,3-dimethyl-2-oxo-butyl)-
3-(2-Carboxy-2-oxo-ethyl)-7-ethyl-1,2-dihydro-4-methyl-
3-(2-Ethoxycarbonyl-2-oxo-ethyl)-7-ethyl-1,2-dihydro-4-methyl-
7-Ethyl-1,2-dihydro-4-methyl-3-(2-oximinopropyl)-
7-Ethyl-1,2-dihydro-4-methyl-3-(3,3-dimethyl-2-oximinobutyl)-
7-Ethyl-1,2-dihydro-4-methyl-3-(2-O-methyl-2-oximino-propyl)-
7-Ethyl-1,2-dihydro-4-methyl-3-(3,3-dimethyl-2-O-methyloximino-butyl)-
7-Ethyl-1,2-dihydro-3-hydroxymethyl-4-methyl-.

**Beispiel 2**

(a) Analog Beispiel 1 erhält man aus 7-Ethyl-1,2-dihydro-4-methyl-2-oxo-chinolin und 4-Brommethyl-2'-(2-triphenylmethyl-2H-5-tetrazolyl)-biphenyl das 7-Ethyl-1,2-dihydro-4-methyl-2-oxo-1-[2'-(2-triphenylmethyl-2H-5-tetrazolyl)-biphenylyl-4-methyl]-chinolin, F. 165°.

(b) Das nach (a) erhaltene Produkt (1 g) wird mit 10 ml Ameisensäure und 90 ml Methanol 2 Std. bei 50° gerührt. Man dampft die erhaltene Lösung ein, arbeitet wie üblich auf und erhält 7-Ethyl-1,2-dihydro-4-methyl-2-oxo-1-[2'-(1H-5-tetrazolyl)-biphenylyl-4-methyl]-chinolin, F. 261°.

**Beispiel 3**

Analog Beispiel 1 erhält man aus 1,87 g 7-Ethyl-1,2-dihydro-4-methyl-2-oxo-chinolin und 2,98 g 3-(p-Brommethyl-phenyl)-2-phenyl-acrylnitril [F. 178°; erhältlich durch Kondensation von p-Tolylaldehyd mit Phenylacetonitril zu 2-Phenyl-3-p-tolylacrylnitril (F. 61°) und Bromierung mit N-Bromsuccinimid in Dichlormethan] das 1-[p-(2-Cyan-2-phenyl-vinyl)-benzyl]-7-ethyl-1,2-dihydro-4-methyl-2-oxo-chinolin, F. 227°.

**Beispiel 4**

Ein Gemisch von 2,52 g 1-p-Aminobenzyl-1,2,3,4-tetrahydro-2-oxo-chinolin (erhältlich durch Reaktion von 1,2,3,4-Tetrahydro-2-oxo-chinolin mit p-Nitrobenzylbromid zu 1,2,3,4-Tetrahydro-1-p-nitrobenzyl-2-oxo-chinolin und anschließende Hydrierung), 1,48 g Phthalsäureanhydrid und 50 ml Chloroform wird 16 Stunden bei 20° gerührt. Das ausgefallene 1-[4-(2-Carboxybenzamido)-benzyl]-1,2,3,4-tetrahydro-2-oxo-chinolinwird abfiltriert.

**Beispiel 5**

Ein Gemisch von 2,52 g 1-p-Aminobenzyl-1,2,3,4-tetrahydro-2-oxo-chinolin, 3 ml Triethylamin, 0,5 g 4-Dimethylaminopyridin und 120 ml Dichlormethan wird auf 5° gekühlt und tropfenweise mit einer Lösung von 2,88 g o-Trifluormethansulfonamidobenzoylchlorid in 20 ml Dichlormethan versetzt. Man rührt noch 16 Stunden bei 20° dampft ein, arbeitet wie üblich auf und erhält 1,2,3,4-Tetranydro-2-oxo-1-[4-(2-trifluormethansulfonamidobenzamido)-benzyl]-chinolin.

**Beispiel 6**

Ein Gemisch von 2,81 g 1-p-Carboxybenzyl-1,2,3,4-tetrahydro-2-oxo-chinolin, 10 g Thionylchlorid und 35 ml Chloroform wird 6 Stunden gekocht und eingedampft. Das erhaltene rohe Säurechlorid wird durch mehrfaches Lösen in Toluol und Eindampfen von Thionylchlorid-Resten befreit und in 80 ml THF gelöst. Man tropft diese Lösung zu einer Lösung von 1,7 g Anthranilsäure und 0,8 g NaOH in 100 ml Wasser, rührt 24 Stunden und säuert mit Salzsäure bis pH 5 an. Nach üblicher Aufarbeitung erhält man 1-[p-(2-Carboxyanilinocarbonyl)-benzyl]-1,2,3,4-tetranydro-2-oxo-chinolin

**Beispiel 7**

Ein Gemisch von 1 g 1,2,3,4-Tetrahydro-1-[4-(α-methoxycarbonyl-benzyloxy)-benzyl]-2-oxo-chinolin (erhältlich durch Reaktion von 1,2,3,4-Tetrahydro-2-oxo-chinolin mit p-Benzyloxy-benzylbromid zum 1-p-Benzyloxybenzylderivat, Hydrogenolyse zu 1-p-Hydroxybenzyl-1,2,3,4-tetrahydro-2-oxo-chinolin und Veretherung mit α-Brom-phenylessigsäure-methylester), 12 ml wässeriger 2 n NaOH-Lösung und 48 ml Ethanol wird 2 Stunden gekocht und dann eingedampft. Man erhält wie üblich auf (wässerige Salzsäure bis pH 3/Dichlormethan) und erhält 1-[4-(α-Carboxy-benzyloxy)-benzyl]-1,2,3,4-tetrahydro-2-oxo-chinolin.

**Beispiel 8**

Eine Lösung von 1 g 7-Ethyl-1,2-dihydro-4-methyl-3-o-nitro-benzyl-2-oxo-1-[2'-(1H-5-tetrazolyl)-biphenylyl-4-methyl]-chinolin in 25 ml Ethanol wird an 0,3 g 5%ig. Pd-Kohle bei 20° und Normaldruck bis zur Aufnahme der berechneten $H_2$-Menge hydriert. Man filtriert den Katalysator ab, dampft ein und reinigt das erhaltene 3-o-Aminobenzyl-7-ethyl-1,2-dihydro-4-methyl-2-oxo-1-[2'-(1H-5-tetrazolyl)-biphenylyl-4-methyl]-chinolin chromatographisch.

Analog erhält man durch Hydrierung der entsprechenden Nitroverbindungen die nachstehenden 1-(2'-Cyan-biphenylyl-4-methyl)-2-oxo-chinoline:
3-o-Aminobenzyl-7-ethyl-1,2-dihydro-4-methyl-
3-m-Aminobenzyl-7-ethyl-1,2-dihydro-4-methyl-
3-p-Aminobenzyl-7-ethyl-1,2-dihydro-4-methyl-
sowie die nachstehenden 2-Oxo-1-[2'-(1H-5-tetrazolyl)-biphenylyl-4-methyl]-chinoline:
3-m-Aminobenzyl-7-ethyl-1,2-dihydro-4-methyl-
3-p-Aminobenzyl-7-ethyl-1,2-dihydro-4-methyl-, F. 166°.

## Beispiel 9

Ein Gemisch von 483 mg 3-o-Aminobenzyl-1-(2'-cyan-biphenylyl-4-methyl)-7-ethyl-1,2-dihydro-4-methyl-2-oxo-chinolin, 200 mg $NaBH_4$ und 10 ml THF wird unter Rühren bei 10-30° tropfenweise mit einer Lösung von 0,3 ml 36%iger wässeriger HCHO-Lösung, 150 mg $H_2SO_4$, 0,5 ml Wasser und 5 ml THF versetzt. Man rührt 16 Std. bei 20°, macht mit KOH alkalisch, arbeitet wie üblich auf und erhält (1-(2'-Cyan-biphenylyl-4-methyl)-3-o-dimethylaminobenzyl-7-ethyl-1,2-dihydro-4-methyl-2-oxo-chinolin.

## Beispiel 10

Ein Gemisch von 434 mg 1-(2'-Cyan-biphenylyl-4-methyl)-7-ethyl-1,2-dihydro-4-methyl-2-oxo-3-(2-oxo-propyl)-chinolin, 167 mg O-Methyl-hydroxylamin-hydrochlorid, 0,16 ml Pyridin und 25 ml Ethanol wird 48 Std. bei 20° gerührt. Man dampft ein, arbeitet wie üblich auf und erhält 1-(2'-Cyan-biphenylyl-4-methyl)-7-ethyl-1,2-dihydro-4-methyl-3-(2-O-methyl-oximino-propyl)-2-oxo-chinolin.

Die nachstehenden Beispiele betreffen pharmazeutische Zubereitungen, die Wirkstoffe der Formel I oder ihre Salze enthalten.

## Beispiel A: Tabletten und Dragees

In üblicher Weise werden Tabletten folgender Zusammensetzung gepreßt, die bei Bedarf mit einer üblichen Drageedecke auf Sucrosegrundlage überzogen werden:

| | |
|---|---|
| Wirkstoff der Formel I | 100 mg |
| Mikrokristalline Cellulose | 278,8 mg |
| Lactose | 110 mg |
| Maisstärke | 11 mg |
| Magnesiumstearat | 5 mg |
| Feinteiliges Siliciumdioxid | 0,2 mg |

## Beispiel B: Hartgelatine-Kapseln

Übliche zweiteilige Hartgelatine-Kapseln werden jeweils gefüllt mit

| | |
|---|---|
| Wirkstoff der Formel I | 100 mg |
| Lactose | 150 mg |
| Cellulose | 50 mg |
| Magnesiumstearat | 6 mg |

## Beispiel C: Weichgelatine-Kapseln

Übliche Weichgelatine-Kapseln werden mit einem Gemisch aus jeweils 50 mg Wirkstoff und 250 mg Olivenöl gefüllt.

**Beispiel D: Ampullen**

Eine Lösung von 200 g Wirkstoff in 2 kg 1,2-Propandiol wird mit Wasser auf 10 l aufgefüllt und in Ampullen gefüllt, so daß jede Ampulle 20 mg Wirkstoff enthält.

**Beispiel E: Wässerige Suspension für orale Applikation**

Eine wässerige Suspension wird in üblicher Weise hergestellt. Die Einheitsdosis (5 ml) enthält 100 mg Wirkstoff, 100 mg Na-Carboxymethylcellulose, 5 ml Na-Benzoat und 100 mg Sorbit.

## Patentansprüche

**1.** 2-Oxochinolinderivate der Formel I

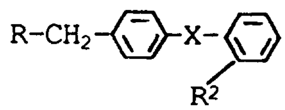

I

worin

R

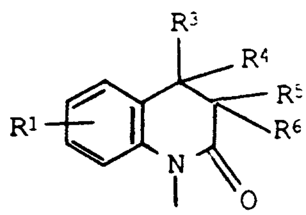

,

| | |
|---|---|
| $R^1$ | H, A, OA oder SA, |
| $R^2$ | H, COOH, COOA, CN, $NO_2$, $NH_2$, $NHCOR^7$, $NHSO_2R^7$ oder 1H-5-Tetrazolyl, |
| $R^3$ und $R^4$ | jeweils H oder A, |
| $R^5$ und $R^6$ | jeweils H, A, COOH, COOA, Cyan-alkyl, HOOC-alkyl, AOOC-alkyl, $H_2NCO$-alkyl, ANHCO-alkyl, $A_2NCO$-alkyl, Cyan-alkenyl, HOOC-alkenyl, AOOC-alkenyl, 1H-5-Tetrazolyl-alkyl, 2-Oxo-oxazolidinyl-alkyl, Ar-alkyl, AO-alkyl, ArO-alkyl, Ar-alkyl-O-alkyl, Formyl-alkyl, Oxo-alkyl, HOOC-oxo-alkyl, AOOC-oxo-alkyl, Oximino-alkyl, O-Alkyl-oximino-alkyl, HO-alkyl oder $R^8R^9N$-alkyl, |
| $R^3$ und $R^5$ | zusammen auch eine Bindung, |
| $R^7$ | Alkyl mit 1-6 C-Atomen, worin auch ein oder mehrere H-Atom(e) durch F ersetzt sein können, |
| $R^8$ | H oder A, |
| $R^9$ | H, A, A-CO, Ar-CO, COOA, $CONH_2$, CONHA, CONHAr, $CON(A)_2$ oder CONAAr, |
| X | fehlt, -NH-CO-, -CO-NH-, -O-CH(COOH)-, -NH-CH(COOH)-, -NA-CH(COOH)-, -CH=C-(COOH)-, -CH=C(CN)- oder -CH=C(1H-5-tetrazolyl)-, |
| A | Alkyl mit 1-6 C-Atomen, |
| Ar | eine unsubstituierte oder eine durch $R^7$, $OR^7$, Hal, COOH, COOA, CN, $NO_2$, $NH_2$, NHA, $N(A)_2$, $NHCOR^7$, $NHSO_2R^7$ oder 1H-5-Tetrazolyl monosubstituierte Phenylgruppe und |
| Hal | F, Cl, Br oder I bedeuten und |

worin der "alkyl"-Teil oder "alkenyl"-Teil der genannten Gruppen jeweils bis zu 6 C-Atome enthält, sowie ihre Salze.

**2.**

a) 1,2-Dihydro-2-oxo-1-[2'-(1H-5-tetrazolyl)-biphenylyl-4-methyl]-chinolin;

b) 1,2,3,4-Tetrahydro-2-oxo-1-[2'-(1H-5-tetrazolyl)-biphenylyl-4-methyl]-chinolin;
c) 7-Ethyl-1,2-dihydro-4-methyl-2-oxo-1-[2'-(1H-5-tetrazolyl)-biphenylyl-4-methyl]-chinolin;
d) 1,2,3,4-Tetrahydro-4,4-dimethyl-2-oxo-1-[2'-(1H-5-tetrazolyl)-biphenylyl-4-methyl]-chinolin;
e) 7-Ethyl-1,2,3,4-tetrahydro-4,4-dimethyl-2-oxo-1-[2'-(1H-5-tetrazolyl)-biphenylyl-4-methyl]-chinolin.

**3.** Verfahren zur Herstellung von 2-Oxo-chinolinderivaten der Formel I nach Anspruch 1 sowie ihrer Salze, dadurch gekennzeichnet, daß man
(a) eine Verbindung der Formel II

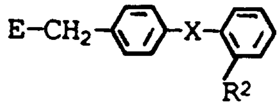

II

worin
E Cl, Br, I oder eine freie oder reaktionsfähig funktionell abgewandelte OH-Gruppe bedeutet und
$R^2$ und X die in Anspruch 1 angegebene Bedeutung haben,
mit einer Verbindung der Formel III

H-R III

worin
R die in Anspruch 1 angegebene Bedeutung hat,
umsetzt
oder
(b) zur Herstellung einer Verbindung der Formel I,
worin
X -NH-CO- oder -CO-NH- bedeutet,
eine Verbindung der Formel IV worin

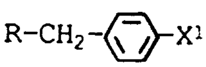

IV

$X^1$ $NH_2$ oder COOH bedeutet und
R die in Anspruch 1 angegebene Bedeutung hat
oder ein reaktionsfähiges Derivat dieser Verbindung mit einer Verbindung der Formel V

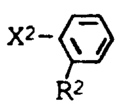

V

worin
$X^2$ COOH (falls $X^1$ $NH_2$ ist) oder $NH_2$ (falls $X^1$ COOH ist) bedeutet und
$R^2$ die in Anspruch 1 angegebene Bedeutung hat,
oder mit einem reaktionsfähigen Derivat dieser Verbindung umsetzt oder
(c) daß man eine Verbindung der Formel I aus einem ihrer funktionellen Derivate durch Behandeln mit einem solvolysierenden oder hydrogenolysierenden Mittel in Freiheit setzt,
und/oder daß man in einer Verbindung der Formel I einen oder mehrere Rest(e) R und/oder $R^2$ in einen oder mehrere andere Reste R und/oder $R^2$ umwandelt und/oder eine Base oder Säure der Formel I in eines ihrer Salze umwandelt.

4. Verfahren zur Herstellung pharmazeutischer Zubereitungen, dadurch gekennzeichnet, daß man eine Verbindung der Formel I nach Anspruch 1 und/oder eines ihrer physiologisch unbedenklichen Säureadditionssalze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel I nach Anspruch 1 und/oder einem ihrer physiologisch unbedenklichen Säureadditionssalze.

6. Verbindung der Formel I nach Anspruch 1 und ihre physiologisch unbedenklichen Säureadditionssalze zur Bekämpfung von Krankheiten.

7. Verwendung von Verbindungen der Formel I nach Anspruch 1 und/oder ihrer physiologisch unbedenklichen Säureadditionssalze zur Herstellung eines Arzneimittels.

8. Verwendung von Verbindungen der Formel I nach Anspruch 1 und/oder ihrer physiologisch unbedenklichen Säureadditionssalze bei der Bekämpfung von Krankheiten.

Europäisches Patentamt

# EUROPÄISCHER RECHERCHENBERICHT

Nummer der Anmeldung

EP 93 10 3710
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | WO-A-9 119 697 (MEIJ SEIKA KABISHIKI KAISHA)<br>26. Dezember 1991 | 1-8 | C07D215/227<br>A61K31/47 |
| P,Y | & EP-A-0 487 745 (MEIJI SEIKA KABUSHIKI KAISHA)<br>3. Juni 1992<br>* Beispiele 72, 74, 76, 92, 94, 96, 98, 100 *<br>* Seite 3, Zeile 36 - Zeile 48; Ansprüche 1,11-13,17-25 *<br>--- | 1-8 | |
| Y | EP-A-0 411 766 (MERCK & CO., INC.)<br>6. Februar 1991<br>* Beispiele *<br>* Seite 3, Zeile 35 - Zeile 37; Ansprüche 1-7 *<br>--- | 1-8 | |
| Y | EP-A-0 187 322 (OTSUKA PHARMACEUTICAL CO., LTD.)<br>16. Juli 1986<br>* Beispiele *<br>* Seite 3, Zeile 1 - Zeile 16; Ansprüche 1-17 *<br>--- | 1-8 | RECHERCHIERTE SACHGEBIETE (Int. Cl.5) |
| Y | EP-A-0 419 048 (MERCK & CO., INC.)<br>27. März 1991<br>* Beispiele *<br>* Seite 3, Zeile 36 - Zeile 38; Ansprüche 1-9 *<br>--- | 1-8 | C07D<br>A61K |
| Y | CHEMICAL ABSTRACTS, vol. 96, no. 21, 24. Mai 1982, Columbus, Ohio, US; abstract no. 181163f, 'Carbostyril derivatives'<br>Seite 701 ;<br>* Zusammenfassung *<br>& JP-A-82 002 274 (Otsuka Pharmaceutical Co., Ltd.; 07-01-1982)<br>---<br>-/-- | 1-8 | |

Der vorliegende Recherchenbericht wurde für alle Patentansprüche erstellt

| Recherchenort | Abschlußdatum der Recherche | Prüfer |
|---|---|---|
| MUENCHEN | 12 MAI 1993 | HERZ C.P. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 93 10 3710
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y | CHEMICAL ABSTRACTS, vol. 97, no. 12, 20. September 1982, Columbus, Ohio, US; abstract no. 98357x, 'Cardiotonic formulations containing aminoalkoxydihydrocarbostyrils' Seite 400 ; * Zusammenfassung * & JP-A-82 080 322 (Otsuka Pharmaceutical Co., Ltd.; 19-05-1982) --- | 1-8 | |
| Y | CHEMICAL ABSTRACTS, vol. 98, no. 5, 31. Januar 1983, Columbus, Ohio, US; abstract no. 34510e, 'Carbostyril derivatives and a cardiotonic composition containing them' Seite 643 ; * Zusammenfassung * & BE-A-892 148 (Otsuka Pharmaceutical Co., Ltd.; 16-08-1982) --- | 1-8 | RECHERCHIERTE SACHGEBIETE (Int. Cl.5 ) |
| Y | CHEMICAL ABSTRACTS, vol. 98, no. 10, 7. März 1983, Columbus, Ohio, US; abstract no. 78131m, 'Synthesis and cardiotonic formulations of carbostyril derivatives' Seite 346 ; * Zusammenfassung * & JP-A-57 154 129 (Otsuka Pharmaceutical Co., Ltd; 22-09-1982) --- | 1-8 | |
| Y | CHEMICAL ABSTRACTS, vol. 100, no. 9, 27. Februar 1984, Columbus, Ohio, US; abstract no. 68187e, 'Carbostyril derivatives as cardiotonics' Seite 589 ; * Zusammenfassung * & JP-A-58 148 817 (Otsuka Pharmaceutical Co., Ltd.; 05-09-1983) ----- | 1-8 | |

Der vorliegende Recherchenbericht wurde für alle Patentansprüche erstellt

| Recherchenort | Abschlußdatum der Recherche | Prüfer |
|---|---|---|
| MUENCHEN | 12 MAI 1993 | HERZ C.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument